# EUROPEAN PATENT SPECIFICATION

(11) **EP 3 062 794 B1**
(45) Date of publication and mention of the grant of the patent: **26.09.2018**
(21) Application number: 14811971.2
(22) Date of filing: 30.10.2014
(51) Int. Cl.: A61K 31/43, A61K 31/439, A61K 31/546, A61P 31/04

(54) **PHARMACEUTICAL COMPOSITIONS COMPRISING ANTIBACTERIAL AGENTS**
PHARMAZEUTISCHE ZUSAMMENSETZUNGEN ENTHALTEND ANTIBAKTERIELLE SUBSTANZEN
COMPOSITIONS PHARMACEUTIQUES COMPRENANT DES COMPOSÉS ANTIBACTÉRIENS

(30) Priority: 30.10.2013 IN 3421MU2013
(43) Date of publication of application: 07.09.2016
(73) Proprietor: Wockhardt Limited, Aurangabad 431006 (IN)
(72) Inventor: BHAGWAT, Sachin, Aurangabad Maharashtra 431005 (IN); PATEL, Mahesh Vithalbhai, Aurangabad Maharashtra 431003 (IN)
(74) Representative: Nederlandsch Octrooibureau
(86) International application number: PCT/IB2014/065708
(87) International publication number: WO 2015/063714

(56) References cited:
- WO-A1-2013/014496
- WO-A1-2014/033560
- US-A1- 2013 225 554

## Description

### FIELD OF THE INVENTION

The invention relates to antibacterial compositions and their use in methods for treating or preventing bacterial infections.

### BACKGROUND OF THE INVENTION

Bacterial infections continue to remain one of the major causes contributing towards human diseases. One of the key challenges in treatment of bacterial infections is the ability of bacteria to develop resistance to one or more antibacterial agents over time. Examples of such bacteria that have developed resistance to typical antibacterial agents include: Penicillin-resistant *Streptococcus pneumoniae,* Vancomycin-resistant *Enterococci,* and Methicillin-resistant *Staphylococcus aureus.* The problem of emerging drug-resistance in bacteria is often tackled by switching to newer antibacterial agents, which can be more expensive and sometimes more toxic. Additionally, this may not be a permanent solution as the bacteria often develop resistance to the newer antibacterial agents as well in due course. In general, bacteria are particularly efficient in developing resistance, because of their ability to multiply very rapidly and pass on the resistance genes as they replicate.

Treatment of infections caused by resistant bacteria remains a key challenge for the clinician community. One example of such challenging pathogen is *Acinetobacter baumannii* (*A. baumannii*), which continues to be an increasingly important and demanding species in healthcare settings. The multidrug resistant nature of this pathogen and its unpredictable susceptibility patterns make empirical and therapeutic decisions more difficult. *A. baumannii* is associated with infections such as pneumonia, bacteremia, wound infections, urinary tract infections and meningitis.

Therefore, there is a need for development of newer ways to treat infections that are becoming resistant to known therapies and methods. Surprisingly, it has been found that compositions comprising a beta-lactam compound selected from cefepime or a pharmaceutically acceptable salt thereof and certain nitrogen containing bicyclic compounds exhibit unexpectedly synergistic antibacterial activity, even against highly resistant bacterial strains.

WO2013/014496A1 discloses an antibacterial effect of cefepime combined with the sodium salt of trans-7-oxo-6-(sulphooxy)-1,6-diazabicyclo-[3.2.1]-octane-2-carboxamide against certain *A baumanni* strains.

US2013/0225554A1 discloses 7-oxo-6-(sulfooxy)-1,6-diazabicyclo [3.2.1] octane-2-carboxamide derivatives and their use as antibacterial agents, either alone or in combination with an antibiotic, for the treatment of infections caused by β-lactamase-producing pathogenic bacteria.

WO2014/033560A1) discloses 7-oxo-6-(sulfooxy)-1,6-diazabicyclo [3.2.1] octane-2-carboxamide derivatives and their use as antibacterial agents, either alone or in combination with a further antibacterial agent, for the treatment of infections caused by β-lactamase-producing pathogenic bacteria.

### SUMMARY OF THE INVENTION

Accordingly, there are provided pharmaceutical compositions comprising: (a) a beta-lactam compound selected from cefepime or a pharmaceutically acceptable salt thereof, and (b) a compound of Formula (I) or a stereoisomer or a pharmaceutically acceptable salt thereof, 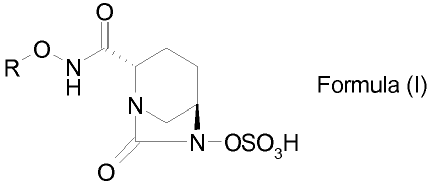 wherein:
R is -(CH₂)ₙ-R₁;
R₁ is pyrrolidine or piperidine;
n is 1;
wherein the compound of Formula (I) or the stereoisomer or the pharmaceutically acceptable salt thereof is present in the composition in an amount from about 0.25 gram to about 4 gram per gram of the beta-lactam compound selected from cefepime or the pharmaceutically acceptable salt thereof.

In one general aspect, pharmaceutical compositions according to the invention are used in treatment or prevention of a bacterial infection.

In another general aspect, there is provided a combination of: (a) a beta-lactam compound selected from cefepime or a pharmaceutically acceptable salt thereof, and (b) a compound of Formula (I) or a stereoisomer or a pharmaceutically acceptable salt thereof for use in a method of treatment or prevention of a bacterial infection in a subject, said method comprising administering said combination to said subject, wherein the amount of the compound of Formula (I) or the stereoisomer or the pharmaceutically acceptable salt thereof administered is from about 0.25 gram to about 4 gram per gram of the beta-lactam compound selected from cefepime or the pharmaceutically acceptable salt thereof.

In another general aspect, there is provided a compound of Formula (I) or a stereoisomer or a pharmaceutically acceptable salt thereof: 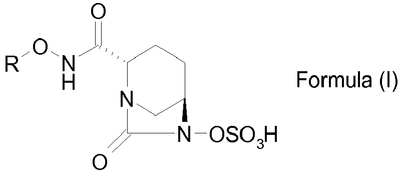 wherein:
R is -(CH₂)ₙ-R₁;
R₁ is pyrrolidine or piperidine;
n is 1;
for use in a method for increasing antibacterial effectiveness of a beta-lactam compound selected from cefepime or a pharmaceutically acceptable salt thereof, said method comprising co-administering the beta-lactam compound with the compound of Formula (I) or the stereoisomer or the pharmaceutically acceptable salt thereof, wherein the amount of the compound of Formula (I) or the stereoisomer or the pharmaceutically acceptable salt thereof administered is from about 0.25 gram to about 4 gram per gram of the beta-lactam compound or the pharmaceutically acceptable salt thereof.

The details of one or more embodiments of the inventions are set forth in the description below. Other features, objects and advantages of the inventions will be apparent from the following description including claims.

### DETAILED DESCRIPTION OF THE INVENTION

Reference will now be made to the exemplary embodiments, and specific language will be used herein to describe the same. It must be noted that, as used in this specification and the appended claims, the singular forms "a", "an", and "the" include plural referents unless the content clearly dictates otherwise.

The inventors have surprisingly discovered that a pharmaceutical composition comprising: (a) a beta-lactam compound selected from cefepime or a pharmaceutically acceptable salt thereof, and (b) a compound of Formula (I) or a stereoisomer or a pharmaceutically acceptable salt thereof, wherein the compound of Formula (I) or the stereoisomer or the pharmaceutically acceptable salt thereof is present in the composition in an amount from about 0.25 gram to about 4 gram per gram of the beta-lactam compound selected from cefepime or the pharmaceutically acceptable salt thereof, exhibits unexpectedly improved antibacterial efficacy, even against highly resistant bacteria, including those producing extended spectrum beta-lactamase enzymes (ESBLs).

The term "infection" or "bacterial infection" as used herein includes presence of bacteria, in or on a subject, which, if its growth were inhibited, would result in a benefit to the subject. As such, the term "infection" in addition to referring to the presence of bacteria also refers to presence of other floras, which are not desirable. The term "infection" includes infection caused by bacteria.

The term "treat", "treating" or "treatment" as used herein refers to administration of a medicament, including a pharmaceutical composition, or one or more pharmaceutically active ingredients, for prophylactic and/or therapeutic purposes. The term "prophylactic treatment" refers to treating a subject who is not yet infected, but who is susceptible to, or otherwise at a risk of infection (preventing the bacterial infection). The term "therapeutic treatment" refers to administering treatment to a subject already suffering from infection. The terms "treat", "treating" or "treatment" as used herein also refer to administering compositions, or one or more of pharmaceutically active ingredients discussed herein, with or without additional pharmaceutically active or inert ingredients, in order to: (i) reduce or eliminate either a bacterial infection, or one or more symptoms of a bacterial infection, or (ii) retard progression of a bacterial infection, or one or more symptoms of a bacterial infection, or (iii) reduce severity of a bacterial infection, or one or more symptoms of a bacterial infection, or (iv) suppress clinical manifestation of a bacterial infection, or (v) suppress manifestation of adverse symptoms of a bacterial infection.

The terms "pharmaceutically effective amount" or "therapeutically effective amount" or "effective amount" as used herein refer to an amount, which has a therapeutic effect or is the amount required to produce a therapeutic effect in a subject. For example, a "therapeutically effective amount" or "pharmaceutically effective amount" or "effective amount" of an antibacterial agent or a pharmaceutical composition is the amount of the antibacterial agent or the pharmaceutical composition required to produce a desired therapeutic effect as may be judged by clinical trial results, model animal infection studies, and/or in vitro studies (e.g. in agar or broth media). Such effective amount depends on several factors, including but not limited to, the microorganism (e.g. bacteria) involved, characteristics of the subject (for example height, weight, sex, age and medical history), severity of infection and particular type of the antibacterial agent used. For prophylactic treatments, a prophylactically effective amount is that amount which would be effective in preventing the bacterial infection.

The term "administration" or "administering" refers to and includes delivery of a composition, or one or more pharmaceutically active ingredients to a subject, including for example, by any appropriate method, which serves to deliver the composition or its active ingredients or other pharmaceutically active ingredients to the site of infection. The method of administration may vary depending on various factors, such as for example, the components of the pharmaceutical composition or type/nature of the pharmaceutically active or inert ingredients, site of the potential or actual infection, the microorganism involved, severity of the infection, age and physical condition of the subject. Some examples of ways to administer a composition or a pharmaceutically active ingredient to a subject according to this invention include oral, intravenous, topical, intrarespiratory, intraperitoneal, intramuscular, parenteral, sublingual, transdermal, intranasal, aerosol, intraocular, intratracheal, intrarectal, vaginal, gene gun, dermal patch, eye drop and mouthwash. In case of a pharmaceutical composition comprising more than one ingredients (active or inert), one of the ways of administering such composition is by admixing the ingredients (e.g. in the form of a suitable unit dosage form such as tablet, capsule, solution or powder) and then administering the dosage form. Alternatively, the ingredients may also be administered separately (simultaneously or one after the other) as long as these ingredients reach beneficial therapeutic levels such that the composition as a whole provides a synergistic and/or desired effect.

The term "growth" as used herein refers to a growth of one or more microorganisms and includes reproduction or population expansion of the microorganism (e.g. bacteria). The term "growth" also includes maintenance of on-going metabolic processes of the microorganism, including the processes that keep the microorganism alive.

The term "effectiveness" as used herein refers to ability of a treatment, or a composition, or one or more pharmaceutically active ingredients to produce a desired biological effect in a subject. For example, the term "antibacterial effectiveness" of a composition or of an antibacterial agent refers to the ability of the composition or the antibacterial agent to prevent or treat bacterial infection in a subject.

The term "synergistic" or "synergy" as used herein refers to the interaction of two or more agents so that their combined effect is greater than their individual effects.

The term "antibacterial agent" as used herein refers to any substance, compound, a combination of substances, or a combination of compounds capable of: (i) inhibiting, reducing or preventing growth of bacteria; (ii) inhibiting or reducing ability of a bacteria to produce infection in a subject; or (iii) inhibiting or reducing ability of bacteria to multiply or remain infective in the environment. The term "antibacterial agent" also refers to compounds capable of decreasing infectivity or virulence of bacteria.

The term "beta-lactam compound" as used herein refers to cefepime.

The term "beta-lactamase" or "beta-lactamase enzyme" as used herein refers to any enzyme or protein or any other substance that breaks down a beta-lactam ring. The term "beta-lactamase" includes enzymes that are produced by bacteria and have the ability to hydrolyse the beta-lactam ring in a beta-lactam compound, either partially or completely.

The term "extended spectrum beta-lactamase" (ESBL) as used herein includes those beta-lactamase enzymes, which are capable of conferring bacterial resistance to various beta-lactam antibacterial agents such as penicillins, cephalosporins and aztreonam.

The term "beta-lactamase inhibitor" as used herein refers to a compound capable of inhibiting activity of one or more beta-lactamase enzymes, either partially or completely.

The term "colony forming units" or "CFU" as used herein refers to an estimate of number of viable bacterial cells per ml of the sample. Typically, a "colony of bacteria" refers to a mass of individual bacteria growing together.

The term "pharmaceutically inert ingredient" or "carrier" or "excipient" refers to and includes compounds or materials used to facilitate administration of a compound, for example, to increase the solubility of the compound. Typical examples of solid carriers include starch, lactose, dicalcium phosphate, sucrose, and kaolin. Typical examples of liquid carriers include sterile water, saline, buffers, non-ionic surfactants, and edible oils. In addition, various adjuvants commonly used in the art may also be included. These and other such compounds are described in literature, e.g., in the Merck Index (Merck & Company, Rahway, N.J.). Considerations for inclusion of various components in pharmaceutical compositions are described, e.g., in Gilman et al. (Goodman and Gilman's: The Pharmacological Basis of Therapeutics, 8th Ed., Pergamon Press., 1990).

The term "subject" as used herein refers to vertebrate or invertebrate, including a mammal. The term "subject" includes human, animal, a bird, a fish, or an amphibian. Typical examples of a "subject" include humans, cats, dogs, horses, sheep, bovine cows, pigs, lambs, rats, mice and guinea pigs.

The term "pharmaceutically acceptable derivative" as used herein refers to and includes any pharmaceutically acceptable salt, pro-drug, metabolite, ester, ether, hydrate, polymorph, solvate, complex, and adduct of a compound described herein which, upon administration to a subject, is capable of providing (directly or indirectly) the parent compound. For example, the term "antibacterial agent or a pharmaceutically acceptable derivative thereof' includes all derivatives of the antibacterial agent (such as salts, pro-drugs, metabolites, esters, ethers, hydrates, polymorphs, solvates, complexes, and adducts) which, upon administration to a subject, are capable of providing (directly or indirectly) the antibacterial agent.

The term "pharmaceutically acceptable salt" as used herein refers to one or more salts of a given compound which possesses desired pharmacological activity of the free compound and which is neither biologically nor otherwise undesirable. In general, the term "pharmaceutically acceptable salts" refer to salts that are suitable for use in contact with the tissues of human and animals without undue toxicity, irritation and allergic response, and are commensurate with a reasonable benefit/risk ratio. Pharmaceutically acceptable salts are well known in the art. For example, S. M. Berge, et al. (J. Pharmaceutical Sciences, 66; 1-19, 1977) describes various pharmaceutically acceptable salts in details.

The term "stereoisomer" as used herein refers to and includes isomeric molecules that have the same molecular formula but differ in positioning of atoms and/or functional groups in the space. Stereoisomers may further be classified as enantiomers (where different isomers are mirror-images of each other) and diastereomers (where different isomers are not mirror-images of each other). Diastereomers include isomers such as conformers, meso compounds, cis-trans (E-Z) isomers, and non-enantiomeric optical isomers.

The term "heterocycloalkyl" as used herein refers to four to seven member cycloalkyl group containing one or more heteroatoms selected from nitrogen, oxygen or sulfur. The heterocycloalkyl group optionally incorporates one or more double or triple bonds, or a combination of double bonds and triple bonds, but which is not aromatic. Typical examples of heterocycloalkyl groups include pyrrolidine, 2-oxo-pyrrolidine, imidazolidin-2-one, piperidine, oxazine, thiazine, piperazine, piperazin-2,3-dione, morpholine, thiamorpholine and azapane. The heterocycloalkyl may be unsubstituted, or substituted with one or more substituents. Typical examples of such substituents include C₁-C₆ alkyl, halogen, alkoxy, CN, COOH, CONH₂, OH, NH₂, NHCOCH₃, heterocyclyl, heteroaryl, aryl, SO₂-alkyl, SO₂-aryl, OSO₂-alkyl and OSO₂-aryl. The term "heterocyclyl" as used herein refers to cyclic groups in which a ring portion includes at least one heteroatom such as oxygen, nitrogen or sulfur. Heterocyclic groups include "heteroaryl" as well as "heterocycloalkyl". The term "heteroaryl" as used herein refers to a monocyclic or polycyclic aromatic hydrocarbon group wherein one or more carbon atoms have been replaced with heteroatoms selected from nitrogen, oxygen or sulfur. Typical examples of heteroaryl group include pyridine, pyrimidine, pyrazine, pyridazine, furan, pyrrol and thiophene. The term "aryl" as used herein refers to a monocyclic or polycyclic aromatic hydrocarbon. Typical examples of aryl groups include phenyl, naphthyl, anthracenyl, fluorenyl and phenanthrenyl.

A person of skills in the art would appreciate that various compounds described herein (including, for example a compound of Formula (I) and cefepime) can exist and are often used as their pharmaceutically acceptable salts.

In a general aspect, there are provided pharmaceutical compositions comprising: (a) a beta-lactam compound selected from cefepime or a pharmaceutically acceptable salt thereof, and (b) a compound of Formula (I) or a stereoisomer or a pharmaceutically acceptable salt thereof: 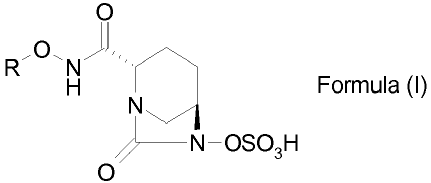 wherein:
R is -(CH₂)ₙ-R₁;
R₁ is pyrrolidine or piperidine;
n is 1;
wherein the compound of Formula (I) or the stereoisomer or the pharmaceutically acceptable salt thereof is present in the composition in an amount from about 0.25 gram to about 4 gram per gram of the beta-lactam compound selected from cefepime or the pharmaceutically acceptable salt thereof.

Compounds of Formula (I), according to the invention can be used in various forms including as such, a stereoisomer or a pharmaceutically acceptable salt thereof. Typical examples of the compound of Formula (I) include the following:
(2S, 5R)-7-oxo-N-[(2S)-pyrrolidin-2-ylmethyloxy]-6-(sulfooxy)-1,6-diazabicyclo [3.2.1] octane-2-carboxamide;
Sulphuric acid, mono[(1R,2S,5R)-7-oxo-2-[[[(2S)-2-pyrrolidinylmethoxy]amino]carbonyl]-1,6-diazabicyclo[3.2.1]oct-6-yl] ester (CAS Registry Number: 1452459-04-9);
Sulphuric acid, mono[(2S,5R)-7-oxo-2-[[[(2S)-2-pyrrolidinylmethoxy]amino]carbonyl]-1,6-diazabicyclo[3.2.1]oct-6-yl]ester (CAS Registry Number: 1572987-32-6);
Sulphuric acid, mono[(2S,5R)-7-oxo-2-[[[(2S)-2-pyrrolidinylmethoxy]amino]carbonyl]-1,6-diazabicyclo[3.2.1]oct-6-yl]ester, sodium salt (1:1)CAS Registry Number: 1572988-44-3;
(2S,5R)-7-oxo-N-[(2S)-piperidine-2-ylmethyloxy]-6-(sulfooxy)-1,6-diazabicyclo [3.2.1] octane-2-carboxamide;
Sulphuric acid, mono[(1R,2S,5R)-7-oxo-2-[[[(2S)-2-piperidinyl methoxy]amino]carbonyl]-1,6-diazabicyclo[3.2.1]oct-6-yl]ester (CAS Registry Number: 1452459-12-9);
Sulphuric acid, mono[(2*S*,5*R*)-7-oxo-2-[[[(2*S*)-2-piperidinylmethoxy]amino]carbonyl]-1,6-diazabicyclo[3.2.1]oct-6-yl]ester (CAS Registry Number: 1572987-74-6);
Sulphuric acid, mono[(2*S*,5*R*)-7-oxo-2-[[[(2S)-2-piperidinylmethoxy]amino]carbonyl]-1,6-diazabicyclo[3.2.1]oct-6-yl]ester, sodium salt (1:1) (CAS Registry Number: 1572988-89-6);
or a stereoisomer or a pharmaceutically acceptable salt thereof.

The compound of Formula (I) may be used in the form of its stereoisomer or a pharmaceutically acceptable salt thereof. Typical examples of suitable pharmaceutically acceptable salts of a compound of Formula (I) include sodium or potassium salts.

In some embodiments, there are provided pharmaceutical compositions comprising: (a) a beta-lactam compound selected from cefepime or a pharmaceutically acceptable salt thereof, and (b) a compound of Formula (I) selected from:
"(2S, 5R)-7-oxo-N-[(2S)-pyrrolidin-2-ylmethyloxy]-6-(sulfooxy)-1,6-diazabicyclo [3.2.1] octane-2-carboxamide";
"(2S,5R)-7-oxo-N-[(2S)-piperidine-2-ylmethyloxy]-6-(sulfooxy)-1,6-diazabicyclo [3.2.1] octane-2-carboxamide";
or a stereoisomer or a pharmaceutically acceptable salt thereof, wherein the compound of Formula (I) or the stereoisomer or the pharmaceutically acceptable salt thereof is present in the composition in an amount from about 0.25 gram to about 4 gram per gram of the beta-lactam compound selected from cefepime or the pharmaceutically acceptable salt thereof.

The active ingredients according to this invention (cefepime or a compound of Formula (I)) may be used in their free forms or in the form of their pharmaceutically acceptable salts.

In one general aspect, there are provided pharmaceutical compositions comprising: (a) a beta-lactam compound selected from cefepime or a pharmaceutically acceptable salt thereof, and (b) a compound of Formula (I) or a stereoisomer or a pharmaceutically acceptable salt thereof; wherein the compound of Formula (I) or the stereoisomer or the pharmaceutically acceptable salt thereof is present in the composition in an amount from about 0.25 gram to about 4 gram per gram of the beta-lactam compound selected from cefepime or the pharmaceutically acceptable salt thereof.

Individual amounts of the compound of Formula (I) or the stereoisomer or the pharmaceutically acceptable salt thereof, and the beta-lactam compound (selected from cefepime or the pharmaceutically acceptable salt thereof) in the composition may vary depending on clinical requirements. In some embodiments, the compound of Formula (I) or the stereoisomer or the pharmaceutically acceptable salt thereof in the composition is present in an amount from about 0.01 gram to about 10 gram. In some other embodiments, the beta-lactam compound selected from cefepime or the pharmaceutically acceptable salt thereof in the composition is present in an amount from about 0.01 gram to about 10 gram.

In some embodiments, the pharmaceutical composition according to the invention comprises about 1 gram of the compound of Formula (I) or the stereoisomer or the pharmaceutically acceptable salt thereof, and about 2 gram of the beta-lactam compound selected from cefepime or the pharmaceutically acceptable salt thereof.

In some other embodiments, the pharmaceutical composition according to the invention comprises about 2 gram of the compound of Formula (I) or the stereoisomer or the pharmaceutically acceptable salt thereof, and about 2 gram of the beta-lactam compound selected from cefepime or the pharmaceutically acceptable salt thereof.

In some embodiments, the pharmaceutical composition according to the invention comprises about 0.5 gram of the compound of Formula (I) or the stereoisomer or the pharmaceutically acceptable salt thereof, and about 2 gram of the beta-lactam compound selected from cefepime or the pharmaceutically acceptable salt thereof.

In some embodiments, the pharmaceutical composition according to the invention comprises about 0.25 gram of the compound of Formula (I) or the stereoisomer or the pharmaceutically acceptable salt thereof, and about 1 gram of the beta-lactam compound selected from cefepime or the pharmaceutically acceptable salt thereof.

In some embodiments, the pharmaceutical composition according to the invention comprises about 0.5 gram of the compound of Formula (I) or the stereoisomer or the pharmaceutically acceptable salt thereof, and about 1 gram of the beta-lactam compound selected from cefepime or the pharmaceutically acceptable salt thereof.

In some embodiments, the pharmaceutical composition according to the invention comprises about 1 gram of the compound of Formula (I) or the stereoisomer or the pharmaceutically acceptable salt thereof, and about 1 gram of the beta-lactam compound selected from cefepime or the pharmaceutically acceptable salt thereof.

In some embodiments, the pharmaceutical composition according to the invention comprises about 2 gram of the compound of Formula (I) or the stereoisomer or the pharmaceutically acceptable salt thereof, and about 1 gram of the beta-lactam compound selected from cefepime or the pharmaceutically acceptable salt thereof.

In some other embodiments, the pharmaceutical composition according to the invention comprises about 1 gram of the compound of Formula (I) or the stereoisomer or the pharmaceutically acceptable salt thereof, and about 0.5 gram of the beta-lactam compound selected from cefepime or the pharmaceutically acceptable salt thereof.

The pharmaceutical composition according to the invention use active as well as inactive ingredients. The active ingredients consist of: (a) a compound of formula (I) or a stereoisomer or a pharmaceutically acceptable salt thereof, and (b) a beta-lactam compound selected from cefepime or a pharmaceutically acceptable salt thereof. The pharmaceutical compositions according to the invention may include one or more pharmaceutically acceptable inactive ingredients such as carriers or excipients. Typical examples of such carriers or excipients include mannitol, lactose, starch, magnesium stearate, sodium saccharine, talcum, cellulose, sodium crosscarmellose, glucose, gelatine, sucrose, magnesium carbonate, wetting agents, emulsifying agents, solubilizing agents, buffering agents, lubricants, preservatives, stabilizing agents and binding agents.

The pharmaceutical compositions or the active ingredients according to the present invention may be formulated into a variety of dosage forms, such as solid, semi-solid, liquid and aerosol dosage forms. Typical examples of some dosage forms include tablets, capsules, powders, solutions, suspensions, suppositories, aerosols, granules, emulsions, syrups and elixirs.

In some embodiments, pharmaceutical compositions according to the invention are in the form of a powder or a solution. In some other embodiments, pharmaceutical compositions according to the invention are present in the form of a powder or a solution that can be reconstituted by addition of a compatible reconstitution diluent prior to administration. In some other embodiments, pharmaceutical compositions according to the invention are in the form of a frozen composition that can be diluted with a compatible reconstitution diluent prior to administration. A typical example of a suitable compatible reconstitution diluent includes water.

In some other embodiments, pharmaceutical compositions according to the invention are present in the form ready to use for parenteral administration.

The compositions according to the invention can be formulated into various dosage forms wherein the active ingredients and/or excipients may be present either together (e.g. as an admixture) or as separate components. When the various ingredients in the composition are formulated as a mixture, such compositions can be delivered by administering such a mixture to a subject using any suitable route of administration. Alternatively, pharmaceutical compositions according to the invention may also be formulated into a dosage form wherein one or more ingredients (such as active or inactive ingredients) are present as separate components. The composition or dosage form wherein the ingredients do not come as a mixture, but come as separate components, such composition/dosage form may be administered in several ways. In one possible way, the ingredients may be mixed in the desired proportions and the mixture is reconstituted in suitable reconstitution diluent and is then administered as required. Alternatively, the components or the ingredients (active or inert) may be separately administered (simultaneously or one after the other) in appropriate proportion so as to achieve the same or equivalent therapeutic level or effect as would have been achieved by administration of the equivalent mixture.

In some embodiments, pharmaceutical compositions according to the invention are formulated into a dosage form such that the compound of Formula (I) or the stereoisomer or the pharmaceutically acceptable salt thereof, and the beta-lactam compound selected from cefepime or the pharmaceutically acceptable salt thereof, are present in the composition as admixture or as a separate components. In some other embodiments, pharmaceutical compositions according to the invention are formulated into a dosage form such that the compound of Formula (I) or the stereoisomer or the pharmaceutically acceptable salt thereof, and the beta-lactam compound selected from cefepime or the pharmaceutically acceptable salt thereof, are present in the composition as separate components.

In one general aspect, pharmaceutical compositions according to the invention are used in treatment or prevention of a bacterial infection.

In case of dosage forms wherein the compound of Formula (I) or the stereoisomer or the pharmaceutically acceptable salt thereof, and the beta-lactam compound selected from cefepime or the pharmaceutically acceptable salt thereof are present in the composition as separate components, the compound of Formula (I) or the stereoisomer or the pharmaceutically acceptable salt thereof may be administered before, after or simultaneously with the administration of the beta-lactam compound selected from cefepime or the pharmaceutically acceptable salt thereof.

In yet another general aspect, there is provided a combination of: (a) a beta-lactam compound selected from cefepime or a pharmaceutically acceptable salt thereof, and (b) a compound of Formula (I) or a stereoisomer or a pharmaceutically acceptable salt thereof: 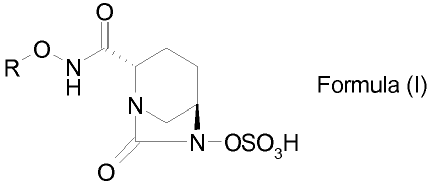 wherein:
R is -(CH₂)ₙ-R₁;
R₁ is pyrrolidine or piperidine;
n is 1;
for use in a method of treatment or prevention of a bacterial infection in a subject, said method comprising administering said combination to said subject, wherein the amount of the compound of Formula (I) or the stereoisomer or the pharmaceutically acceptable salt thereof administered is from about 0.25 gram to about 4 gram per gram of the beta-lactam compound selected from cefepime or the pharmaceutically acceptable salt thereof.

In some embodiments, there is provided a combination of: (a) a beta-lactam compound selected from cefepime or a pharmaceutically acceptable salt thereof, and (b) a compound of Formula (I) or a stereoisomer or a pharmaceutically acceptable salt thereof, for use in a method of treatment or prevention of a bacterial infection in a subject, said method comprising administering said combination to said subject in any of the following amounts:
(i) about 1 gram of the compound of Formula (I) or the stereoisomer or the pharmaceutically acceptable salt thereof, and about 2 gram of the beta-lactam compound selected from cefepime or the pharmaceutically acceptable salt thereof;
(ii) about 2 gram of the compound of Formula (I) or the stereoisomer or the pharmaceutically acceptable salt thereof, and about 2 gram of the beta-lactam compound selected from cefepime or the pharmaceutically acceptable salt thereof;
(iii) about 0.5 gram of the compound of Formula (I) or the stereoisomer or the pharmaceutically acceptable salt thereof, and about 2 gram of the beta-lactam compound selected from cefepime or the pharmaceutically acceptable salt thereof;
(iv) about 0.25 gram of the compound of Formula (I) or a stereoisomer or a pharmaceutically acceptable salt thereof, and about 1 gram of the beta-lactam compound selected from cefepime or the pharmaceutically acceptable salt thereof;
(v) about 0.5 gram of the compound of Formula (I) or the stereoisomer or the pharmaceutically acceptable salt thereof, and about 1 gram of the beta-lactam compound selected from cefepime or the pharmaceutically acceptable salt thereof;
(vi) about 1 gram of the compound of Formula (I) or the stereoisomer or the pharmaceutically acceptable salt thereof, and about 1 gram of the beta-lactam compound selected from cefepime or the pharmaceutically acceptable salt thereof;
(vii) about 2 gram of the compound of Formula (I) or the stereoisomer or the pharmaceutically acceptable salt thereof, and about 1 gram of the beta-lactam compound selected from cefepime or the pharmaceutically acceptable salt thereof; or
(viii) about 1 gram of the compound of Formula (I) or the stereoisomer or the pharmaceutically acceptable salt thereof, and about 0.5 gram of the beta-lactam compound selected from cefepime or the pharmaceutically acceptable salt thereof.

In some embodiments, the compound of Formula (I) or the stereoisomer or the pharmaceutically acceptable salt thereof is administered in an amount from about 0.01 gram to about 10 gram. In some other embodiments, the beta-lactam compound selected from cefepime or the pharmaceutically acceptable salt thereof is administered in an amount from about 0.01 gram to about 10 gram.

In some embodiments, the compound of Formula (I) or the stereoisomer or the pharmaceutically acceptable salt thereof is administered before, after or simultaneously with the administration of the beta-lactam compound selected from cefepime or the pharmaceutically acceptable salt thereof.

The pharmaceutical composition and/or other pharmaceutically active ingredients disclosed herein may be administered by any appropriate method, which serves to deliver the composition, or its constituents, or the active ingredients to the desired site. The method of administration can vary depending on various factors, such as for example, the components of the pharmaceutical composition and the nature of the active ingredients, the site of the potential or actual infection, the microorganism (e.g. bacteria) involved, severity of infection, age and physical condition of the subject. Some examples of administering the composition to a subject according to this invention include oral, intravenous, topical, intrarespiratory, intraperitoneal, intramuscular, parenteral, sublingual, transdermal, intranasal, aerosol, intraocular, intratracheal, intrarectal, vaginal, gene gun, dermal patch, eye drop, ear drop or mouthwash. In some embodiments, the compositions or one or more active ingredients according to the invention are administered parenterally.

In some embodiments, in the compositions and combinations for use according to the invention, a compound of Formula (I) is:
(a) "(2S, 5R)-7-oxo-N-[(2S)-pyrrolidin-2-ylmethyloxy]-6-(sulfooxy)-1,6-diazabicyclo [3.2.1] octane-2-carboxamide";
(b) "(2S,5R)-7-oxo-N-[(2S)-piperidine-2-ylmethyloxy]-6-(sulfooxy)-1,6-diazabicyclo [3.2.1] octane-2-carboxamide";
or a stereoisomer or a pharmaceutically acceptable salt thereof.

In some embodiments, in compositions and combinations for use according to the invention, the compound of Formula (I) is present as a sodium or potassium salt.

In some embodiments, there is provided a compound of Formula (I) or a stereoisomer or a pharmaceutically acceptable salt thereof: 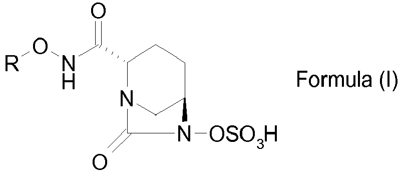 wherein:
R is -(CH₂)ₙ-R₁;
R₁ is pyrrolidine or piperidine;
n is 1;
for use in a method for increasing antibacterial effectiveness of a beta-lactam compound selected from cefepime or a pharmaceutically acceptable salt thereof, said method comprising co-administering the beta-lactam compound with the compound of Formula (I) or the stereoisomer or the pharmaceutically acceptable salt thereof, wherein the amount of the compound of Formula (I) or the stereoisomer or the pharmaceutically acceptable salt thereof administered is from about 0.25 gram to about 4 gram per gram of the beta-lactam compound or the pharmaceutically acceptable salt thereof.

A wide variety of bacterial infections can be treated or prevented using compositions according to the invention. Typical examples of bacterial infections that can be treated or prevented using pharmaceutical compositions and combinations according to the invention include *E. coli* infections, *Yersinia pestis* (pneumonic plague), staphylococcal infection, mycobacteria infection, bacterial pneumonia, *Shigella* dysentery, *Serratia* infections, *Candida* infections, *Cryptococcal* infection, anthrax, tuberculosis or infections caused by *Pseudomonas aeruginosa, Acinetobacter baumannii* or methicillin resistant *Staphylococcus aurues* (MRSA).

The pharmaceutical compositions according to the invention are useful in treatment or prevention of several infections, including for example, skin and soft tissue infections, febrile neutropenia, urinary tract infection, intraabdominal infections, respiratory tract infections, pneumonia (nosocomial), bacteremia meningitis and surgical infections.

In some embodiments, pharmaceutical compositions and combinations according to the invention are used in treatment or prevention of infections caused by resistant bacteria. In some other embodiments, the compositions and combinations according to the invention are used in treatment or prevention of infections caused by bacteria producing one or more beta-lactamase enzymes.

In general, the pharmaceutical compositions and methods disclosed herein are also effective in preventing or treating infections caused by bacteria that are considered to be less or not susceptible to one or more of known antibacterial agents or their known compositions. Some examples of such bacteria known to have developed resistance to various antibacterial agents include *Acinetobacter, Escherichia coli, Pseudomonas aeruginosa, Staphylococcus aureus, Enterobacter, Klebsiella* and *Citrobacter.*

### EXAMPLES

The following examples illustrate the embodiments of the invention that are presently best known. While the present invention has been described above with particularity, the following examples provide further detail in connection with what are presently deemed to be the most practical and preferred embodiments of the invention.

The broth microdilution Minimum Inhibitory Concentration (MIC) studies were conducted as per the CLSI recommendations (Clinical and Laboratory Standards Institute (CLSI), Performance Standards for Antimicrobial Susceptibility Testing, 20th Informational Supplement, M 100-S20, Volume 30, No. 1, 2010). The media utilized was Brain Heart Infusion broth (BD, USA). In brief, 50 mcl of serial double dilutions of the antibacterial agents in Brain Heart Infusion media were dispensed in 96 well microtiter plate. 50 mcl of culture suspension was added to each pre dispensed drug dilution such that the final bacterial inoculum density was 2-8 × 10⁵ CFU/ml. The plates were incubated for 20-24 hours at 35 ± 2° C and observed visually for growth or no growth based on the turbidity. Three specific compounds generally represented by a general Formula (I) were used and are as follows:
1. (2S, 5R)-7-oxo-N-[(3S)-pyrrolidin-3-yloxy]-6-(sulfooxy)-1,6-diazabicyclo[3.2.1] octane -2-carboxamide (**Compound A,** not according to the invention), 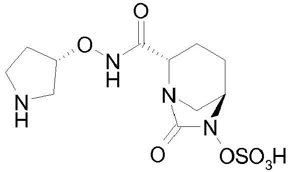
2. (2S, 5R)-7-oxo-N-[(2S)-pyrrolidin-2-ylmethyloxy]-6-(sulfooxy)-1,6-diazabicyclo [3.2.1] octane-2-carboxamide (**Compound B**, according to the invention) and 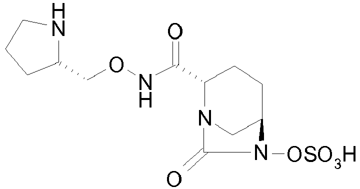
3. (2S,5R)-7-oxo-N-[(2S)-piperidine-2-ylmethyloxy]-6-(sulfooxy)-1,6-diazabicyclo [3.2.1] octane-2- carboxamide (**Compound C,** according to the invention).

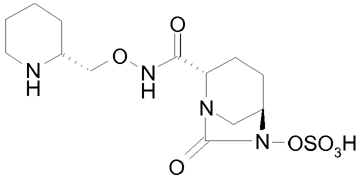

The results of antibacterial activity of various compounds when used alone and in combinations according to the inventions are shown in Tables 1 and 2 respectively. The strains of *A. baumannii* used for the study were those which produced carbapenem hydrolyzing class D beta-lactamase enzymes (OXA 23, OXA 24 or OXA 27). As can be seen from Table 1, that Compound A, Compound B and Compound C when used alone did not exhibit any antibacterial activity (MIC: > 128 mcg/ml). Also, Sulbactam, Cefepime and Meropenem when used alone exhibited poor antibacterial activity against *A. baumannii.* However, surprisingly, as can be seen from the Table 2, the combination according to the invention, exhibited synergistic antibacterial activity against *A. baumannii.* In general, the MIC values of Sulbactam and Cefepime reduced when these were combined with Compounds A, B and C.

Thus the results in Tables 1-2, clearly and surprisingly demonstrate synergistic antibacterial potential of combinations according to invention against *Acinetobacter baumannii* strains harbouring Class D beta-lactamase resistance mechanisms.

| **Table 1. Antibacterial Activity of various compounds against *A. baumannii*** | | | | | | | | | | | | | |
|---|---|---|---|---|---|---|---|---|---|---|---|---|---|
| **Sr.** | **Strain** | | **Enzymes** | | **MIC (mcg/ml)** | | | | | | | | |
| | | | | | **Sulbactam** | **Cefepime** | **Meropenem** | | **Compound A** | | **Compound B** | | **Compound C** |
| 1. | *A. baumannii* NCTC 13301 | | OXA 51, OXA 23 | | 8 | > 32 | 32 | | > 128 | | > 128 | | > 128 |
| 2. | *A. baumannii* NCTC 13302 | | OXA 51, OXA 24 | | 16 | 32 | > 32 | | > 128 | | > 128 | | > 128 |
| 3. | *A. baumannii* NCTC 13304 | | OXA 51, OXA 27 | | 32 | 32 | > 32 | | > 128 | | > 128 | | > 128 |

| **Table 2. Antibacterial activity of sulbactam and Cefepime in combination with compounds A, B and C.** | | | | | | | | | | | | | |
|---|---|---|---|---|---|---|---|---|---|---|---|---|---|
| **Sr.** | **Strain** | **Enzymes** | | **MIC (mcg/ml) of Sulbactam in combination of** | | | | | | **MIC (mcg/ml) of Cefepime in combination of** | | | |
| | | | | **Compound A (4 mcg/ml)** | | **Compound B (4 mcg/ml)** | | **Compound C (4 mcg/ml)** | | **Compound A (8 mcg/ml)** | | **Compound B (8 mcg/ml)** | **Compound C (8 mcg/ml)** |
| 1. | *A. baumannii* NCTC 13301 | OXA 51, OXA 23 | | 4 | | 4 | | 4 | | 16 | | 16 | 32 |
| 2. | *A. baumannii* NCTC 13302 | OXA 51, OXA 24 | | 8 | | 4 | | 8 | | 16 | | 16 | 16 |
| 3. | *A. baumannii* NCTC 13304 | OXA 51, OXA 27 | | 8 | | 4 | | 8 | | 16 | | 8 | 8 |

## Claims

1. A pharmaceutical composition comprising: (a) a beta-lactam compound selected from cefepime or a pharmaceutically acceptable salt thereof, and (b) a compound of Formula (I) or a stereoisomer or a pharmaceutically acceptable salt thereof: 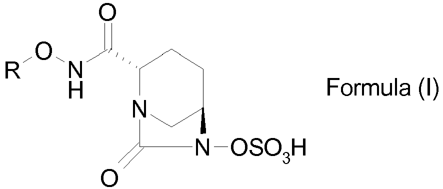 wherein:
R is -(CH₂)ₙ-R₁;
R₁ is pyrrolidine or piperidine;
n is 1;
wherein the compound of Formula (I) or the stereoisomer or the pharmaceutically acceptable salt thereof is present in the composition in an amount from about 0.25 gram to about 4 gram per gram of the beta-lactam compound selected from cefepime or the pharmaceutically acceptable salt thereof.

2. The pharmaceutical composition according to the Claim 1, wherein the compound of Formula (I) or the stereoisomer or the pharmaceutically acceptable salt thereof is present in the composition in an amount from about 0.01 gram to about 10 gram.

3. The pharmaceutical composition according to the Claim 1, wherein the beta-lactam compound selected from cefepime or the pharmaceutically acceptable salt thereof is present in the composition in an amount from about 0.01 gram to about 10 gram.

4. The pharmaceutical composition according to any one of the Claims 1 to 3, comprising: (a) the beta-lactam compound selected from cefepime or the pharmaceutically acceptable salt thereof, and (b) the compound of Formula (I) or the stereoisomer or the pharmaceutically acceptable salt thereof, in any one of the following amounts:
(i) about 1 gram of the compound of Formula (I) or the stereoisomer or the pharmaceutically acceptable salt thereof, and about 2 gram of the beta-lactam compound selected from cefepime or the pharmaceutically acceptable salt thereof;
(ii) about 2 gram of the compound of Formula (I) or the stereoisomer or the pharmaceutically acceptable salt thereof, and about 2 gram of the beta-lactam compound selected from cefepime or the pharmaceutically acceptable salt thereof;
(iii) about 0.5 gram of the compound of Formula (I) or the stereoisomer or the pharmaceutically acceptable salt thereof, and about 2 gram of the beta-lactam compound selected from cefepime or the pharmaceutically acceptable salt thereof;
(iv) about 0.25 gram of the compound of Formula (I) or the stereoisomer or the pharmaceutically acceptable salt thereof, and about 1 gram of the beta-lactam compound selected from cefepime or the pharmaceutically acceptable salt thereof;
(v) about 0.5 gram of the compound of Formula (I) or the stereoisomer or the pharmaceutically acceptable salt thereof, and about 1 gram of the beta-lactam compound selected from cefepime or the pharmaceutically acceptable salt thereof;
(vi) about 1 gram of the compound of Formula (I) or the stereoisomer or the pharmaceutically acceptable salt thereof, and about 1 gram of the beta-lactam compound selected from cefepime or the pharmaceutically acceptable salt thereof;
(vii) about 2 gram of the compound of Formula (I) or the stereoisomer or the pharmaceutically acceptable salt thereof, and about 1 gram of the beta-lactam compound selected from cefepime or the pharmaceutically acceptable salt thereof; or
(viii) about 1 gram of the compound of Formula (I) or the stereoisomer or the pharmaceutically acceptable salt thereof, and about 0.5 gram of the beta-lactam compound selected from cefepime or the pharmaceutically acceptable salt thereof.

5. The pharmaceutical composition according to any one of the Claims 1 to 4, wherein the compound of Formula (I) is: "(2S, 5R)-7-oxo-N-[(2S)-pyrrolidin-2-ylmethyloxy]-6-(sulfooxy)-1,6-diazabicyclo [3.2.1] octane-2-carboxamide" or the stereoisomer or the pharmaceutically acceptable salt thereof.

6. The pharmaceutical composition according to any one of the Claims 1 to 4, wherein the compound of Formula (I) is: "(2S, 5R)-7-oxo-N-[(2S)-piperidine-2-ylmethyloxy]-6-(sulfooxy)-1,6-diazabicyclo [3.2.1] octane-2-carboxamide" or the stereoisomer or the pharmaceutically acceptable salt thereof.

7. A pharmaceutical composition according to any one of the Claims 1 to 6, wherein the composition is formulated into a dosage form such that the compound of Formula (I) or the stereoisomer or the pharmaceutically acceptable salt thereof, and the beta-lactam compound selected from cefepime or the pharmaceutically acceptable salt thereof, are present in the composition as admixture or as separate components.

8. The pharmaceutical composition according to any one of the Claims 1 to 7, wherein the composition is in the form of a powder or a solution.

9. A pharmaceutical composition according to Claim 8, wherein the composition is in the form of a powder or a solution that is suitable for reconstitution by addition of a compatible reconstitution diluent for use in parenteral administration.

10. The pharmaceutical composition according to any one of the Claims 1 to 9 for use in treatment or prevention of a bacterial infection.

11. A combination of: (a) a beta-lactam compound selected from cefepime or a pharmaceutically acceptable salt thereof, and (b) a compound of Formula (I) or a stereoisomer or a pharmaceutically acceptable salt thereof: 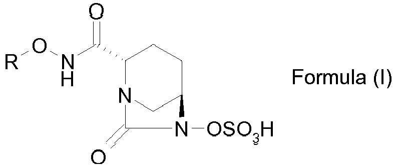 wherein:
R is -(CH₂)ₙ-R₁;
R₁ is pyrrolidine or piperidine;
n is 1;
for use in a method of treatment or prevention of a bacterial infection in a subject, said method comprising administering said combination to said subject; wherein the amount of the compound of Formula (I) or the stereoisomer or the pharmaceutically acceptable salt thereof administered is from about 0.25 gram to about 4 gram per gram of the beta-lactam compound selected from cefepime or the pharmaceutically acceptable salt thereof.

12. The combination for use according to Claim 13, wherein the compound of Formula (I) is selected from any one of the following:
(i) "(2S, 5R)-7-oxo-N-[(2S)-pyrrolidin-2-ylmethyloxy]-6-(sulfooxy)-1,6-diazabicyclo [3.2.1] octane-2-carboxamide",
(ii) "(2S,5R)-7-oxo-N-[(2S)-piperidine-2-ylmethyloxy]-6-(sulfooxy)-1,6-diazabicyclo [3.2.1] octane-2-carboxamide",
or a stereoisomer or a pharmaceutically acceptable salt thereof.

13. A compound of Formula (I) or a stereoisomer or a pharmaceutically acceptable salt thereof: 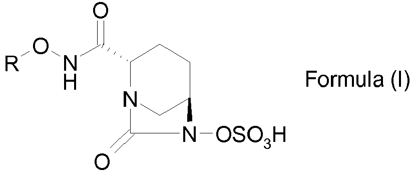 wherein:
R is -(CH₂)ₙ-R₁;
R₁ is pyrrolidine or piperidine;
n is 1;
for use in a method for increasing antibacterial effectiveness of a beta-lactam compound selected from cefepime or a pharmaceutically acceptable salt thereof, said method comprising co-administering the beta-lactam compound with the compound of Formula (I) or the stereoisomer or the pharmaceutically acceptable salt thereof; wherein the amount of the compound of Formula (I) or the stereoisomer or the pharmaceutically acceptable salt thereof administered is from about 0.25 gram to about 4 gram per gram of the beta-lactam compound or the pharmaceutically acceptable salt thereof.

## Patentansprüche

1. Pharmazeutische Zusammensetzung umfassend: (a) eine Beta-Lactamverbindung ausgewählt unter Cefepim oder einem pharmazeutisch akzeptablen Salz davon und (b) eine Verbindung der Formel (I) oder ein Stereoisomer oder ein pharmazeutisch akzeptables Salz davon: 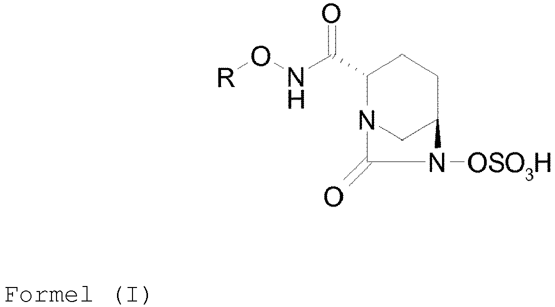 wobei
R -(CH₂)ₙ-R₁ ist;
R₁ Pyrrolidin oder Piperidin ist;
n 1 beträgt;
wobei die Verbindung der Formel (I) oder das Stereoisomer oder das pharmazeutisch akzeptable Salz davon in der Zusammensetzung in einer Menge von etwa 0,25 Gramm bis etwa 4 Gramm pro Gramm der unter Cefepim oder dem pharmazeutisch akzeptablen Salz davon ausgewählten Beta-Lactamverbindung vorliegt.

2. Pharmazeutische Zusammensetzung nach Anspruch 1, wobei die Verbindung der Formel (I) oder das Stereoisomer oder das pharmazeutisch akzeptable Salz davon in der Zusammensetzung in einer Menge von etwa 0,01 Gramm bis etwa 10 Gramm vorliegt.

3. Pharmazeutische Zusammensetzung nach Anspruch 1, wobei die unter Cefepim oder dem pharmazeutisch akzeptablen Salz davon ausgewählte Beta-Lactamverbindung in der Zusammensetzung in einer Menge von etwa 0,01 Gramm bis etwa 10 Gramm vorliegt.

4. Pharmazeutische Zusammensetzung nach irgendeinem der Ansprüche 1 bis 3, umfassend: (a) die Beta-Lactamverbindung ausgewählt unter Cefepim oder dem pharmazeutisch akzeptablen Salz davon und (b) die Verbindung der Formel (I) oder das Stereoisomer oder das pharmazeutisch akzeptable Salz davon in irgendeiner der folgenden Mengen:
(i) etwa 1 Gramm der Verbindung der Formel (I) oder des Stereoisomers oder des pharmazeutisch akzeptablen Salzes davon und etwa 2 Gramm der unter Cefepim oder dem pharmazeutisch akzeptablen Salz davon ausgewählten Beta-Lactamverbindung;
(ii) etwa 2 Gramm der Verbindung der Formel (I) oder des Stereoisomers oder des pharmazeutisch akzeptablen Salzes davon und etwa 2 Gramm der unter Cefepim oder dem pharmazeutisch akzeptablen Salz davon ausgewählten Beta-Lactamverbindung;
(iii) etwa 0,5 Gramm der Verbindung der Formel (I) oder des Stereoisomers oder des pharmazeutisch akzeptablen Salzes davon und etwa 2 Gramm der unter Cefepim oder dem pharmazeutisch akzeptablen Salz davon ausgewählten Beta-Lactamverbindung;
(iv) etwa 0,25 Gramm der Verbindung der Formel (I) oder des Stereoisomers oder des pharmazeutisch akzeptablen Salzes davon und etwa 1 Gramm der unter Cefepim oder dem pharmazeutisch akzeptablen Salz davon ausgewählten Beta-Lactamverbindung;
(v) etwa 0,5 Gramm der Verbindung der Formel (I) oder des Stereoisomers oder des pharmazeutisch akzeptablen Salzes davon und etwa 1 Gramm der unter Cefepim oder dem pharmazeutisch akzeptablen Salz davon ausgewählten Beta-Lactamverbindung;
(vi) etwa 1 Gramm der Verbindung der Formel (I) oder des Stereoisomers oder des pharmazeutisch akzeptablen Salzes davon und etwa 1 Gramm der unter Cefepim oder dem pharmazeutisch akzeptablen Salz davon ausgewählten Beta-Lactamverbindung;
(vii) etwa 2 Gramm der Verbindung der Formel (I) oder des Stereoisomers oder des pharmazeutisch akzeptablen Salzes davon und etwa 1 Gramm der unter Cefepim oder dem pharmazeutisch akzeptablen Salz davon ausgewählten Beta-Lactamverbindung;
(viii) etwa 1 Gramm der Verbindung der Formel (I) oder des Stereoisomers oder des pharmazeutisch akzeptablen Salzes davon und etwa 0,5 Gramm der unter Cefepim oder dem pharmazeutisch akzeptablen Salz davon ausgewählten Beta-Lactamverbindung.

5. Pharmazeutische Zusammensetzung nach irgendeinem der Ansprüche 1 bis 4, wobei die Verbindung der Formel (I):
"(2S,5R)-7-Oxo-N-[(2S)-pyrrolidin-2-ylmethyloxy]-6-(sulfooxy)-1,6-diazabicyclo-[3,2,1]-octan-2-carboxamid" oder das Stereoisomer oder das pharmazeutisch akzeptable Salz davon ist.

6. Pharmazeutische Zusammensetzung nach irgendeinem der Ansprüche 1 bis 4, wobei die Verbindung der Formel (I) "(2S,5R)-7-Oxo-N-[(2S)-piperidin-2-ylmethyloxy]-6-(sulfooxy)-1,6-diazabicyclo-[3,2,1]-octan-2-carboxamid" oder das Stereoisomer oder das pharmazeutisch akzeptables Salz davon ist.

7. Pharmazeutische Zusammensetzung nach irgendeinem der Ansprüche 1 bis 6, wobei die Zusammensetzung in eine Darreichungsform formuliert ist derart, dass die Verbindung der Formel (I) oder das Stereoisomer oder das pharmazeutisch akzeptable Salz davon und die unter Cefepim oder dem pharmazeutisch akzeptablen Salz davon ausgewählte Beta-Lactamverbindung in der Zusammensetzung als Beimischung oder als getrennte Komponenten vorliegen.

8. Pharmazeutische Zusammensetzung nach irgendeinem der Ansprüche 1 bis 7, wobei die Zusammensetzung in Form eines Pulvers oder einer Lösung vorliegt.

9. Pharmazeutische Zusammensetzung nach Anspruch 8, wobei die Zusammensetzung in Form eines Pulvers oder einer Lösung vorliegt, das/die zur Rekonstitution durch Zugabe eines verträglichen Rekonstitutionsverdünnungsmittels zur Verwendung zur parenterale Verabreichung geeignet ist.

10. Pharmazeutische Zusammensetzung nach irgendeinem der Ansprüche 1 bis 9 zur Verwendung bei der Behandlung oder Prävention einer Bakterieninfektion.

11. Kombination von (a) einer Beta-Lactamverbindung ausgewählt unter Cefepim oder einem pharmazeutisch akzeptablen Salz und (b) einer Verbindung der Formel (I) oder einem Stereoisomer oder einem pharmazeutisch akzeptablen Salz davon: 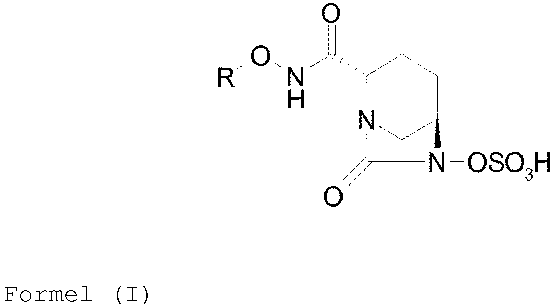 wobei
R -(CH₂)ₙ-R₁ ist;
R₁ Pyrrolidin oder Piperidin ist;
n 1 beträgt;
zur Verwendung bei einem Verfahren zur Behandlung oder Prävention einer Bakterieninfektion bei einem Subjekt, wobei das Verfahren das Verabreichen der Kombination dem Subjekt umfasst; wobei die verabreichte Menge der Verbindung der Formel (I) oder des Stereoisomers oder des pharmazeutisch akzeptablen Salzes davon etwa 0,25 Gramm bis etwa 4 Gramm pro Gramm der unter Cefepim oder dem pharmazeutisch akzeptablen Salz davon ausgewählten Beta-Lactamverbindung beträgt.

12. Kombination zur Verwendung nach Anspruch 13, wobei die Verbindung der Formel (I) von irgendeinem der Folgenden ausgewählt wird:
(i) "(2S,5R)-7-Oxo-N-[(2S)-pyrrolidin-2-ylmethyloxy]-6-(sulfooxy)-1,6-diazabicyclo-[3,2,1]-octan-2-carboxamid",
(ii) "(2S,5R)-7-Oxo-N-[(2S)-piperidin-2-ylmethyloxy]-6-(sulfooxy)-1,6-diazabicyclo-[3,2,1]-octan-2-carboxamid" oder einem Stereoisomer,
oder einem pharmazeutisch akzeptables Salz davon.

13. Verbindung der Formel (I) oder Stereoisomer oder pharmazeutisch akzeptables Salz davon: 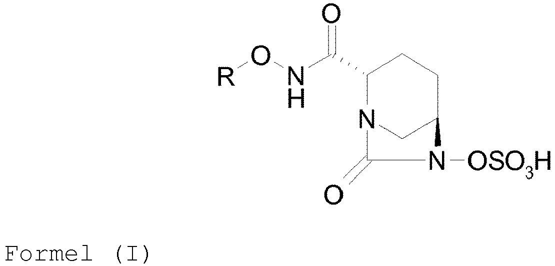 wobei:
R (CH₂)ₙ-R₁ ist;
R₁ Pyrrolidin oder Piperidin ist;
n 1 beträgt;
zur Verwendung bei einem Verfahren zur Erhöhung der antibakteriellen Wirksamkeit einer unter Cefepim oder dem pharmazeutisch akzeptablen Salz davon ausgewählten Beta-Lactamverbindung, wobei das Verfahren das gleichzeitige Verabreichen der Beta-Lactamverbindung mit der Verbindung der Formel (I) oder dem Stereoisomer oder dem pharmazeutisch akzeptablen Salz davon umfasst, wobei die verabreichte Menge der Verbindung der Formel (I) oder des Stereoisomers oder des pharmazeutisch akzeptablen Salzes davon etwa 0,25 Gramm bis etwa 4 Gramm pro Gramm der Beta-Lactamverbindung oder des pharmazeutisch akzeptablen Salzes davon beträgt.

## Revendications

1. Composition pharmaceutique comprenant : (a) un composé bêta-lactame choisi parmi la céfépime ou un sel pharmaceutiquement acceptable de celle-ci, et (b) un composé de Formule (I) ou un stéréoisomère ou un sel pharmaceutiquement acceptable de celui-ci : 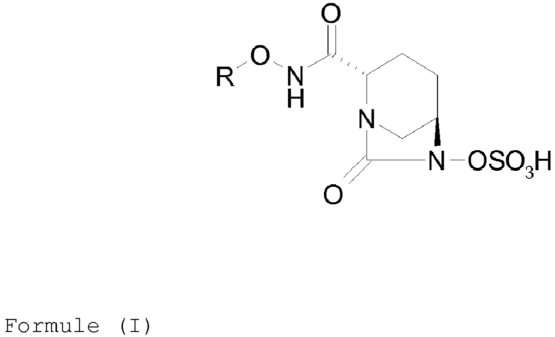 dans laquelle :
R représente -(CH2)n-R1 ;
R1 représente la pyrrolidine ou la pipéridine ;
n vaut 1 ;
dans laquelle le composé de Formule (I) ou le stéréoisomère ou le sel pharmaceutiquement acceptable de celui-ci est présent dans la composition dans une quantité comprise entre environ 0,25 g et environ 4 g par gramme du composé bêta-lactame choisi parmi la céfépime ou le sel pharmaceutiquement acceptable de celle-ci.

2. Composition pharmaceutique selon la revendication 1, dans laquelle le composé de Formule (I) ou le stéréoisomère ou le sel pharmaceutiquement acceptable de celui-ci est présent dans la composition dans une quantité comprise entre environ 0,01 g et environ 10 g.

3. Composition pharmaceutique selon la revendication 1, dans laquelle le composé bêta-lactame choisi parmi la céfépime ou un sel pharmaceutiquement acceptable de celle-ci est présent dans la composition dans une quantité comprise entre environ 0,01 g et environ 10 g.

4. Composition pharmaceutique selon l'une quelconque des revendications 1 à 3, comprenant : (a) le composé bêta-lactame choisi parmi la céfépime ou le sel pharmaceutiquement acceptable de celle-ci, et (b) le composé de Formule (I) ou le stéréoisomère ou le sel pharmaceutiquement acceptable de celui-ci, dans l'une quelconque des quantités suivantes :
(i) environ 1 g du composé de Formule (I) ou du stéréoisomère ou du sel pharmaceutiquement acceptable de celui-ci, et environ 2 g du composé bêta-lactame choisi parmi la céfépime ou le sel pharmaceutiquement acceptable de celle-ci ;
(ii) environ 2 g du composé de Formule (I) ou du stéréoisomère ou du sel pharmaceutiquement acceptable de celui-ci, et environ 2 g du composé bêta-lactame choisi parmi la céfépime ou le sel pharmaceutiquement acceptable de celle-ci ;
(iii) environ 0,5 g du composé de Formule (I) ou du stéréoisomère ou du sel pharmaceutiquement acceptable de celui-ci, et environ 2 g du composé bêta-lactame choisi parmi la céfépime ou le sel pharmaceutiquement acceptable de celle-ci ;
(iv) environ 0,25 g du composé de Formule (I) ou du stéréoisomère ou du sel pharmaceutiquement acceptable de celui-ci, et environ 1 g du composé bêta-lactame choisi parmi la céfépime ou le sel pharmaceutiquement acceptable de celle-ci ;
(v) environ 0,5 g du composé de Formule (I) ou du stéréoisomère ou du sel pharmaceutiquement acceptable de celui-ci, et environ 1 g du composé bêta-lactame choisi parmi la céfépime ou le sel pharmaceutiquement acceptable de celle-ci ;
(vi) environ 1 g du composé de Formule (I) ou du stéréoisomère ou du sel pharmaceutiquement acceptable de celui-ci, et environ 1 g du composé bêta-lactame choisi parmi la céfépime ou le sel pharmaceutiquement acceptable de celle-ci ;
(vii) environ 2 g du composé de Formule (I) ou du stéréoisomère ou du sel pharmaceutiquement acceptable de celui-ci, et environ 1 g du composé bêta-lactame choisi parmi la céfépime ou le sel pharmaceutiquement acceptable de celle-ci ; ou
(viii) environ 1 g du composé de Formule (I) ou du stéréoisomère ou du sel pharmaceutiquement acceptable de celui-ci, et environ 0,5 g du composé bêta-lactame choisi parmi la céfépime ou le sel pharmaceutiquement acceptable de celle-ci.

5. Composition pharmaceutique selon l'une quelconque des revendications 1 à 4, dans laquelle le composé de Formule (I) est : le « (2S,5R)-7-oxo-N-[(2S)-pyrrolidin-2-ylméthyloxy]-6-(sulfooxy)-1,6-diazabicyclo [3.2.1]octane-2-carboxamide » ou le stéréoisomère ou le sel pharmaceutiquement acceptable de celui-ci.

6. Composition pharmaceutique selon l'une quelconque des revendications 1 à 4, dans laquelle le composé de Formule (I) est : le « (2S,5R)-7-oxo-N-[(2S)-pipéridine-2-ylméthyloxy]-6-(sulfooxy)-1,6-diazabicyclo [3.2.1]octane-2-carboxamide » ou le stéréoisomère ou le sel pharmaceutiquement acceptable de celui-ci.

7. Composition pharmaceutique selon l'une quelconque des revendications 1 à 6, dans laquelle la composition est formulée sous une forme posologique telle que le composé de Formule (I) ou le stéréoisomère ou le sel pharmaceutiquement acceptable de celui-ci et le composé bêta-lactame choisi parmi la céfépime ou le sel pharmaceutiquement acceptable de celle-ci, sont présents dans la composition comme un mélange ou comme des composés séparés.

8. Composition pharmaceutique selon l'une quelconque des revendications 1 à 7, dans laquelle la composition est sous la forme d'une poudre ou d'une solution.

9. Composition pharmaceutique selon la revendication 8, dans laquelle la composition est sous la forme d'une poudre ou d'une solution qui est appropriée pour la reconstitution par addition d'un diluant de reconstitution compatible pour une utilisation en administration parentérale.

10. Composition pharmaceutique selon l'une quelconque des revendications 1 à 9 pour une utilisation dans le traitement ou la prévention d'une infection bactérienne.

11. Combinaison : (a) d'un composé bêta-lactame choisi parmi la céfépime ou d'un sel pharmaceutiquement acceptable de celle-ci, et (b) d'un composé de Formule (I) ou d'un stéréoisomère ou d'un sel pharmaceutiquement acceptable de celui-ci : 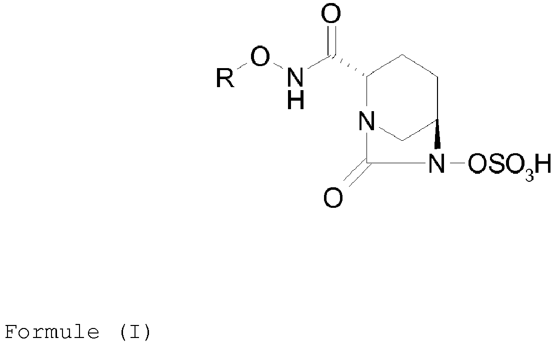 dans laquelle :
R représente -(CH2)n-R1 ;
R1 représente la pyrrolidine ou la pipéridine ;
n vaut 1 ;
pour une utilisation dans un procédé de traitement ou de prévention d'une infection bactérienne chez un sujet, ledit procédé comprenant l'administration de ladite combinaison audit sujet, dans lequel la quantité du composé de Formule (I) ou du stéréoisomère ou du sel pharmaceutiquement acceptable de celui-ci administrée est comprise entre environ 0,25 g et environ 4 g par gramme du composé bêta-lactame choisi parmi la céfépime ou du sel pharmaceutiquement acceptable de celle-ci.

12. Combinaison pour une utilisation selon la revendication 13, dans laquelle le composé de Formule (I) est choisi parmi l'un quelconque des composés suivants :
(i) « (2S,5R)-7-oxo-N-[(2S)-pyrrolidin-2-ylméthyloxy]-6-(sulfooxy)-1,6-diazabicyclo [3.2.1]octane-2-carboxamide »,
(ii) « (2S,5R)-7-oxo-N-[(2S)-pipéridine-2-ylméthyloxy]-6-(sulfooxy)-1,6-diazabicyclo [3.2.1]octane-2-carboxamide »,
ou un stéréoisomère ou un sel pharmaceutiquement acceptable de celui-ci.

13. Composé de Formule (I) ou un stéréoisomère ou un sel pharmaceutiquement acceptable de celui-ci : 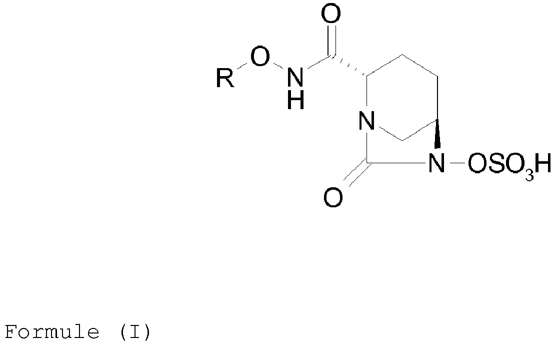 dans laquelle :
R représente -(CH2)n-R1 ;
R1 représente la pyrrolidine ou la pipéridine ;
n vaut 1 ;
pour une utilisation dans un procédé permettant d'augmenter l'efficacité antibactérienne d'un composé bêta-lactame choisi parmi la céfépime ou le sel pharmaceutiquement acceptable de celle-ci, ledit procédé consistant à coadministrer le composé bêta-lactame avec le composé de Formule (I) ou le stéréoisomère ou le sel pharmaceutiquement acceptable de celui-ci ; dans laquelle la quantité du composé de Formule (I) ou du stéréoisomère ou du sel pharmaceutiquement acceptable de celui-ci administrée est comprise entre environ 0,25 g et environ 4 g par gramme du composé bêta-lactame ou du sel pharmaceutiquement acceptable de celle-ci.
